(19) Europäisches Patentamt
European Patent Office
Office européen des brevets

(11) Veröffentlichungsnummer : **0 351 615 B1**

(12) **EUROPÄISCHE PATENTSCHRIFT**

(45) Veröffentlichungstag der Patentschrift : **28.10.92 Patentblatt 92/44**

(51) Int. Cl.$^5$ : **C07C 303/08,** C07C 309/44

(21) Anmeldenummer : **89112038.8**

(22) Anmeldetag : **01.07.89**

# (54) Verfahren zur Herstellung von 4-Alkoxy-2-hydroxybenzophenon-5-sulfonsäuren.

(30) Priorität : **20.07.88 DE 3824486**

(43) Veröffentlichungstag der Anmeldung : **24.01.90 Patentblatt 90/04**

(45) Bekanntmachung des Hinweises auf die Patenterteilung : **28.10.92 Patentblatt 92/44**

(84) Benannte Vertragsstaaten : **CH DE FR GB IT LI**

(56) Entgegenhaltungen :
**FR-A- 853 353**
**US-A- 3 468 938**
**HOUBEN-WEYL, Methoden der Organischen Chemie, Band IX, 1955, Seiten 510-511, herausgegeben von Eugen Müller et al., GeorgThieme Verlag, Stuttgart, DE**

(73) Patentinhaber : **BASF Aktiengesellschaft**
**Carl-Bosch-Strasse 38**
**W-6700 Ludwigshafen (DE)**

(72) Erfinder : **Neumann, Peter, Dr.**
**Poststrasse 28**
**W-6800 Mannheim 31 (DE)**
Erfinder : **Eilingsfeld, Heinz, Dr.**
**Pierstrasse 9 a**
**W-6710 Frankenthal (DE)**
Erfinder : **Aumueller, Alexander, Dr.**
**An der Marlach 5**
**W-6705 Deidesheim (DE)**

EP 0 351 615 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues und verbessertes Verfahren zur Herstellung von 4-Alkoxy-2-hydroxybenzophenon-5-sulfonsäuren der allgemeinen Formel I

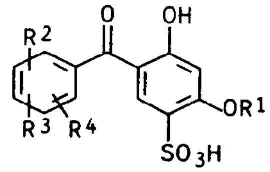

(I),

in der

$R^1$ $C_1$-$C_{20}$-Alkyl, das gegebenenfalls durch Halogen, Cyano, Hydroxy, $C_1$-$C_{20}$-Alkoxy, $C_2$-$C_{20}$-Alkoxycarbonyl, Acyloxy und/oder gegebenenfalls durch $C_1$-$C_4$-Alkyl, $C_1$-$C_4$-Alkoxy und/oder Halogen substituiertes Phenyl, substituiert ist,

$R^2$, $R^3$ unabhängig voneinander Wasserstoff, Halogen, $C_1$-$C_{12}$-Alkyl, $C_1$-$C_{12}$-Alkoxy, $C_1$-$C_4$-Halogenalkyl, $C_3$-$C_8$-Cycloalkyl, $C_4$-$C_{12}$-Cycloalkyl-alkyl, Cyano, Hydroxy, Hydroxyethyl oder gegebenenfalls durch $C_1$-$C_4$-Alkyl, $C_1$-$C_4$-Alkoxy und/oder Halogen substituiertes Phenyl, Phenoxy oder $C_7$-$C_{10}$-Phenalkyl und

$R^4$ Wasserstoff oder eine Sulfonsäuregruppe

bedeuten, durch Umsetzung eines 4-Alkoxy-2-hydroxybenzophenons der allgemeinen Formel II

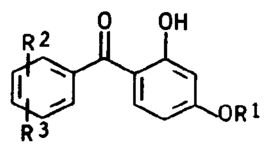

(II),

mit Chlorsulfonsäure bei Temperaturen von (-20) bis +100°C, indem man die Umsetzung in einem aliphatischen, cycloaliphatischen und/oder aromatischen Carbonsäureester als Lösungsmittel vornimmt.

Aus der US-A-3 468 938 und der US-A-3 636 077 ist bekannt, daß 4-Alkoxy-2-hydroxybenzophenone durch Umsetzung mit Chlorsulfonsäure in chlorierten Kohlenwasserstoffen hergestellt werden können. Dieses Verfahren weist jedoch eine Reihe von Nachteilen auf, wie beispielsweise die Toxizität der genannten Lösungsmittel, weshalb solche Reaktionen in geschlossenen Apparaten mit einer aufwendigen Reinigung der Abluft und des Abwassers durchgeführt werden müssen.

Der Erfindung lag die Aufgabe zugrunde, einen neuen und verbesserten Zugang zu den 4-Alkoxy-2-hydroxybenzophenon-5-sulfonsäuren zu finden und den Nachteilen des bekannten Verfahrens abzuhelfen.

Demgemäß wurde ein neues und verbessertes Verfahren zur Herstellung von 4-Alkoxy-2-hydroxybenzophenon-5-sulfonsäuren durch Umsetzung von 4-Alkoxy-2-hydroxybenzophenonen mit Chlorsulfonsäure gefunden, welches dadurch gekennzeichnet ist, daß man die Umsetzung in einem aliphatischen, cycloaliphatischen und/oder aromatischen Carbonsäureester als Lösungsmittel vornimmt.

Die 4-Alkoxy-2-hydroxybenzophenon-5-sulfonsäuren sind nach folgender Methode erhältlich:

Die Umsetzung erfolgt zwischen einem 4-Alkoxy-2-hydroxybenzophenon und Chlorsulfonsäure bei Temperaturen von (-20) bis 100°C unter Verwendung eines aliphatischen, cycloaliphatischen und/oder aromatischen Carbonsäureesters nach folgender Reaktionsgleichung:

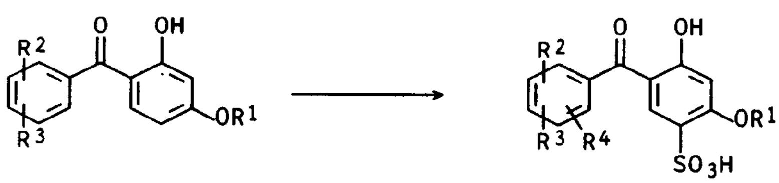

(II) (I)

Das Verfahren kann kontinuierlich oder diskontinuierlich durchgeführt werden.

Die 4-Alkoxy-2-hydroxybenzophenone II sind bekannt und können auf übliche Weise, beispielsweise durch

Friedel-Crafts-Acylierung von Resorcin mit Carbonsäurechloriden der Formel III

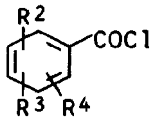

(III),

in der $R^4$ für Wasserstoff steht und anschließender Alkylierung der Hydroxyfunktion nach an sich bekannten Methoden (z.B. DE-PS 1 917 409) hergestellt werden. Tri- und Tetrahydroxybenzophenone bzw. deren Ether werden nach den Verfahren der US-A-2 789 140, US-A-2 686 812, US-A-2 773 903 und US-A-3 073 866 erhalten.

Die Umsetzung kann bei Temperaturen von -20°C bis +100°C und Drücken von 0,01 bis 50 bar erfolgen, vorzugsweise zwischen 0°C und +40°C und 0,1 und 2 bar, besonders bevorzugt bei Normaldruck (ca. 1 bar).

Als Lösungsmittel eignen sich alle Carbonsäureester, die zwischen -20°C und +100°C flüssig sind, wie aliphatische, cycloaliphatische oder aromatische Mono- oder Polycarbonsäureester von aliphatischen Mono- oder Polyalkoholen. Sie sind unter den Reaktionsbedingungen praktisch inert und können bei der Aufarbeitung auf einfache Weise, z.B. durch Destillation, zurückgewonnen und wieder eingesetzt werden.

Anstelle der reinen Carbonsäureester können auch Gemische eingesetzt werden, wie z.B. solche mit techn. Isooctylalkohol, techn. Isononylalkohol, techn. Isodecylalkohol, techn. Isotridecylalkohol oder techn. Isooctydecylalkohol als Alkoholkomponente. Bevorzugt sind $C_1$-$C_4$-Alkylester der Essigsäure, Propionsäure, Benzoesäure oder Phthalsäure, insbesondere der Essigsäureethyl- und -butylester, Benzoesäuremethyl- und -ethylester sowie der Phthalsäuremethyl-, -ethyl- oder -butylester.

Beispielhaft seien die Ester der Carbonsäuren wie Essigsäure, Propionsäure, 2-Methylbuttersäure, Buttersäure, Isobuttersäure, Valeriansäure, Isovaleriansäure, Pivalinsäure, Capronsäure, Önanthsäure, 2-Ethylhexansäure, Caprylsäure, Caprinsäure, Laurinsäure, Myristinsäure, Palmitinsäure, Stearinsäure, Arachinsäure, Behensäure, Lignocerinsäure, Cyclohexancarbonsäure, Oxalsäure, Malonsäure, Bernsteinsäure, Glutarsäure, Adipinsäure, Korksäure, Sebacinsäure, Benzoesäure, 2-, 3- oder 4-Fluorbenzoesäure, 2-, 3- oder 4-Chlorbenzoesäure, 2-, 3- oder 4-Methylbenzoesäure, Phthalsäure, Isophthalsäure, und Terephthalsäure mit Alkoholen wie Methanol, Ethanol, 1-Butanol, Isobutanol, sec-Butanol, tert-Butanol, 1-Pentanol, 2-Methyl-1-butanol, 3-Methyl-1-butanol, 2-Methyl-2-butanol, 2,2-Dimethyl-1-propanol, 1-Hexanol, 2-Ethyl-1-butanol, 2-Hexanol, 1-Heptanol, 1-Octanol, 2-Ethyl-1-hexanol, 3,3-Dimethyl-1-hexanol, 3,5-Dimethyl-1-hexanol, 4,5-Dimethyl-1-hexanol, 1-Nonanol, 3,5,5-Trimethyl-1-hexanol, 1-Decanol, 3,5-Dimethyloctanol, 1-Undecanol, 1-Dodecanol, 1-Tridecanol, 1-Tetradecanol, 1-Pentadecanol, 1-Hexadecanol, 1-Heptadecanol, 1-Octadecanol, 1-Nonadecanol, 1-Eicosanol, 1-Heneicosanol, 1-Docosanol, 1-Tricosanol, 1-Tetracosanol, 1-Pentacosanol, 1-hexacosanal, 1-Heptacosanol, 1-Octacosanol, 1-Nonacosanol, 1-Triacontanol, Cyclohexanol, Ethylenglykol, Glycerin, Propylenglykol, 1,2-Butandiol, 1,4-Butandiol, 2,3-Butandiol, 1,2,4-Butantriol, 1,2-Pentadiol, 2,2-Dimethyl-1,3-propandiol, 1,5-Pentandiol, 1,6-Hexandiol, Pentaerythrit, Trimethylolethan und Trimethylolpropan genannt.

Im einzelnen seien folgende Ester beispielhaft genannt: Methylacetat, Ethylacetat, Propylacetat, Isopropylacetat, Butylacetat, Isobutylacetat, 2-Ethylhexylacetat, Ethylenglykoldiacetat, Glyceryltriacetat, Glyceryltripropionat, Glyceryltripalmitat, Isobutylisobutyarat, Bis(2-ethylhexyl)-adipat, Butylglykolat, Dimethylphthalat, Diethylphthalat, Dibutylphthalat, Bis(2-ethylhexyl)phthalat, Bis(2-ethylhexyl)-terephthalat, Bis(2-ethylhexyl)-trimellitat.

Die Menge des Lösungsmittels kann in weiten Grenzen variieren, z.B. zwischen 500 und 5000 ml pro Mol 4-Alkoxy-2-hydroxybenzophenon II, vorzugsweise zwischen 500 und 2500 ml, besonders bevorzugt von 500 bis 2000 ml.

Bei der erfindungsgemäßen Sulfonierung wird in der Regel eine Sulfosäuregruppe in den 4-Alkoxy-2-hydroxykern der Ausgangsverbindung eingeführt. Ist die Reaktivität des zweiten Kerns durch entsprechende Substitution mit Elektronendonatorgruppen wie Hydroxy, Alkoxy erhöht, so können auch zwei Sulfonsäurereste eingeführt werden, beispielsweise liefert die Umsetzung von 2,2′-Dihydroxy-4,4′-dimethoxybenzophenon mit 2 mol Sulfonierungsreagens die 2,2′-Dihydroxy-4,4′-dimetoxy-5,5′disulfonsäure.

Bei der Monosulfonierung werden Edukt (II) und das Sulfonierungsmittel Chlorsulfonsäure im Molverhältnis von 0,01:1 bis 10:1, vorzugsweise 0,1:1 bis 1,2:1, besonders bevorzugt etwa 1:1 umgesetzt, vorteilhaft ist ein geringer Überschuß (1 bis 20 Mol%) des Sulfonierungsmittels, um eine vollständige Umsetzung zu erreichen.

Die Aufarbeitung des rohen Reaktionsgemisches und Isolierung der Sulfonsäure (I) kann in an sich bekannter Weise erfolgen, z.B. durch Absaugen des gegebenenfalls bei Kühlung nach Reaktionsende ausgefallenen Reaktionsproduktes oder durch Eindampfen des Reaktionsgemisches, gegebenenfalls unter vermindertem Druck. Ein weiteres Aufarbeitungsverfahren besteht darin, daß man das Reaktionsgemisch nach beendeter Umsetzung in vorgelegtes Wasser einlaufen läßt oder es mit Wasser versetzt, in diesem Fall kann eine vorhergehende Kühlung der Reaktionsmischung entfallen.

Aus der wäßrigen Lösung können die Endstoffe (II) dann durch Spühtrocknen, wie in der US-PS 33 468 938 beschrieben, isoliert oder als in Wasser schwer lösliche Alkali- oder Erdalkalisalze oder Ammoniumsalze ausgefällt oder beispielsweise durch Extraktion mit Aminen als Ammoniumsalze isoliert werden.

Die Substituenten in den Formeln I und II haben folgende Bedeutungen:

$R^1$

- unverzweigtes oder verzweigtes $C_1$-$C_{20}$-Alkyl, vorzugsweise unverzweigtes oder verzweigtes $C_1$-$C_{18}$-Alkyl, wie Methyl, Ethyl, n-Propyl, iso-Propyl, n-butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, 1,1,3,3-Tetramethylbutyl, Nonyl, iso-Nonyl, n-Decyl, iso-Decyl, n-Undecyl, iso-Undecyl, n-Dodecyl, iso-Dodecyl, n-Tridecyl, n-Tetradecyl, N-Pentadecyl, n-Hexadecyl, n-Heptadecyl und n-Octadecyl,
- unverzweigtes oder verzweigtes $C_1$-$C_{20}$-Halogenalkyl, vorzugsweise unverzweigtes oder verzweigtes $C_1$-$C_8$-Fluor- oder Chloralkyl, besonders bevorzugt unverzweigtes oder verzweigtes $C_1$-$C_4$-Fluor- und/oder Chloralkyl wie 2-Chlorethyl und 2,2,2-Trifluorethyl,
- unverzweigtes oder verzweigtes $C_1$-$C_{20}$-Cyanoalkyl, vorzugsweise unverzweigtes oder verzweigtes $C_1$-$C_8$-Cyanoalkyl, besonders bevorzugt unverzweigtes oder verzweigtes $C_1$-$C_4$-Cyanoalkyl, wie Cyanomethyl, 1-Cyanoethyl, 2-Cyanoethyl und 4-Cyanbutyl,
- unverzweigtes oder verzweigtes $C_2$-$C_{20}$-Hydroxyalkyl, vorzugsweise unverzweigtes oder verzweigtes $C_2$-$C_8$-Hydroxyalkyl, besonders bevorzugt unverzweigtes oder verzweigtes $C_2$-$C_4$-Hydroxyalkyl, wie 2-Hydroxyethyl, 3-Hydroxypropyl und 4-Hydroxybutyl,
- unverzweigtes oder verzweigtes $C_2$-$C_{40}$-Alkoxyalkyl, vorzugsweise $C_2$-$C_{20}$-Alkoxyalkyl, besonders bevorzugt $C_2$-$C_8$-Alkoxyalkyl wie Methoxymethyl, 1-Methoxyethyl, 2-Methoxyethyl und Ethoxymethyl,
- unverzweigtes oder verzweigtes durch C2-$C_{20}$-Alkoxycarbonyl substituiertes $C_1$-$C_{20}$-Alkyl, vorzugsweise unverzweigtes oder verzweigtes durch $C_2$-$C_8$-Alkoxycarbonyl substituiertes $C_1$-$C_8$-Alkyl, besonders bevorzugt unverzweigtes oder verzweigtes durch $C_2$-$C_5$-Alkoxycarbonyl substituiertes $C_1$-$C_4$-Alkyl, wie Methoxycarbonylmethyl, Ethoxycarbonylmethyl, n-Propoxycarbonylmethyl, Butoxycarbonylmethyl, 2-Methoxycarbonylethyl, 2-Ethoxycarbonylethyl, 2-n-Propoxycarbonylethyl, 2-n-Butoxycarbonylethyl, 3-Methoxycarbonylpropyl, 3-Ethoxycarbonylpropyl, 3-n-Propoxy-carbonylpropyl, 3-Butoxycarbonylpropyl, 4-Methoxycarbonylbutyl, 4-Ethoxycarbonylbutyl, 4-n-Propoxycarbonylbutyl und 4-n-Butylcarbonylbutyl,
- unverzweigtes oder verzweigtes durch $C_1$-$C_{20}$-Acyloxy substituiertes $C_2$-$C_{20}$-Alkyl, vorzugsweise unverzweigtes oder verzweigtes durch Acyloxy substituiertes $C_2$-$C_8$-Alkyl, besonders bevorzugt unverzweigtes oder verzweigtes durch $C_1$-$C_4$-Acyloxy substituiertes $C_2$-$C_4$-Alkyl, wie 2-Acetyloxyethyl, 2-Propanoyloxyethyl, 2-Butanoyloxyethyl, 3-Acetyloxypropyl, 3-Propanoyloxyethyl, 3-Butanoyloxypropyl, 4-Acetyloxybutyl, 4-Propanoyloxybutyl und 4-Butanoyloxybutyl,
- unverzweigtes oder verzweigtes $C_7$-$C_{26}$-Phenalkyl, vorzugsweise unverzweigtes oder verzweigtes $C_7$-$C_{14}$-Phenalkyl, besonders bevorzugt unverzweigtes oder verzweigtes $C_7$-$C_{10}$-Phenalkyl, wie Benzyl, 1-Phenethyl, 2-Phenethyl, 3-Phenpropyl und 4-Phenbutyl,
- unverzweigtes oder verzweigtes im Phenylteil ein- bis dreifach durch $C_1$-$C_4$-Alkyl substituiertes $C_7$-$C_{26}$-Phenalkyl, vorzugsweise unverzweigtes oder verzweigtes im Phenylteil ein- bis dreifach durch $C_1$-$C_4$-Alkyl substituiertes $C_7$-$C_{14}$-Phenalkyl, besonders bevorzugt unverzweigtes oder verzweigtes im Phenylteil durch $C_1$-$C_4$-Alkyl monosubstituiertes $C_7$-$C_{10}$-Phenalkyl, wie 2-Methylbenzyl und 4-Methylbenzyl,
- unverzweigtes oder verzweigtes im Phenylteil ein- bis dreifach durch $C_1$-$C_4$-Alkoxy substituiertes $C_7$-$C_{26}$-Phenalkyl, vorzugsweise unverzweigtes oder verzweigtes im Phenylteil ein- bis dreifach durch $C_1$-$C_4$-Alkoxy substituiertes $C_7$-$C_{14}$-Phenalkyl, besonders bevorzugt unverzweigtes oder verzweigtes durch $C_{1-4}$-Alkoxy monosubstituiertes $C_7$-$C_{10}$-Phenalkyl, wie 2-Methoxybenzyl, 4-Methoxybenzyl, 2-Ethoxybenzyl und 4-Ethoxybenzyl,
- unverzweigtes oder verzweigtes im Phenylteil ein- bis dreifach durch Halogen substituiertes $C_7$-$C_{26}$-Phenalkyl, vorzugsweise unverzweigtes oder verzweigtes im Phenylteil ein- bis dreifach durch Fluor und/oder Chlor substituiertes $C_7$-$C_{14}$-Phenalkyl, besonders bevorzugt unverzweigtes oder verzweigtes im Phenylteil durch Fluor oder Chlor monosubstituiertes $C_7$-$C_{10}$-Phenalkyl, wie 2-Fluorbenzyl, 4-Fluorbenzyl, 2-Chlorbenzyl und 4-Chlorbenzyl,
- unverzweigtes oder verzweigtes im Phenylteil zwei- oder dreifach durch $C_1$-$C_4$-Alkyl und $C_1$-$C_4$-Alkoxy substituiertes $C_7$-$C_{26}$-Phenalkyl, vorzugsweise unverzweigtes oder verzweigtes im Phenylteil zwei- bis dreifach durch $C_1$-$C_4$-Alkyl und $C_1$-$C_4$-Alkoxy substituiertes $C_7$-$C_{10}$-Phenyalkyl, wie 3-Methyl-4-methoxybenzyl und 3,5-Dimethyl-4-methoxybenzyl,

- unverzweigtes oder verzweigtes im Phenylteil zwei- oder dreifach durch $C_1$-$C_4$-Alkyl und Halogen substituiertes $C_7$-$C_{26}$-Phenylalkyl, vorzugsweise unverzweigtes oder verzweigtes im Phenylteil zwei- oder dreifach durch $C_1$-$C_4$-Alkyl und Halogen substituiertes $C_7$-$C_{10}$-Phenylalkyl, wie 3-Chlor-4-methylbenzyl,
- unverzweigtes oder verzweigtes im Phenylteil zwei- oder dreifach durch $C_1$-$C_4$-Alkoxy und Halogen substituiertes $C_7$-$C_{26}$-Phenylalkyl, vorzugsweise unverzweigtes oder verzweigtes im Phenylteil zwei- oder dreifach durch $C_1$-$C_4$-Alkoxy und Halogen substituiertes $C_7$-$C_{10}$-Phenylalkyl, wie 3-Chlor-4-methoxybenzyl,
- unverzweigtes oder verzweigtes im Phenylteil durch $C_1$-$C_4$-Alkyl, $C_1$-$C_4$-Alkoxy mit Halogen substituiertes $C_7$-$C_{26}$-Phenalkyl, vorzugsweise unverzweigtes oder verzweigtes im Phenylteil durch $C_1$-$C_4$-Alkyl, $C_1$-$C_4$-Alkoxy mit Halogen substituiertes $C_7$-$C_{10}$-Phenalkyl, wie 2-Chlor-3-methyl-4-methoxybenzyl,

$R^2$, $R^3$ unabhängig voneinander

- Wasserstoff,
- Halogen, wie Fluor, Chlor, Brom und Iod, vorzugsweise Fluor und Chlor
- unverzweigtes oder verzweigtes $C_1$-$C_{12}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl,
- unverzweigtes oder verzweigtes $C_1$-$C_{12}$-Alkoxy, wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, tert-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, 1,1,3,3-Tetramethylbutoxy, n-Nonoxy, iso-Nonoxy, n-Decoxy, iso-Decoxy, n-Undecoxy, iso-Undecoxy, n-Dodecoxy und iso-Dodecoxy,
- unverzweigtes oder verzweigtes $C_1$-$C_4$-Halogenalkyl, wie 1,2-Dibrom-2,2-dimethylethyl, bevorzugt $C_1$-$C_2$-Fluor- oder Chloralkyl, wie Fluormethyl, Difluormethyl, Trifluormethyl, Chlormethyl, Chlorflormethyl, Dichlorfluormethyl, Chlordifluormethyl, 2,2,2-Trifluorethyl und 2,2,2-Trichlorethyl,
- $C_3$-$C_8$-Cycloalkyl, wie Cyclopropyl, Cyclobutyl, Cyclopentyl, Cyclohexyl, Cycloheptyl und Cyclooctyl, bevorzugt Cyclohexyl,
- $C_4$-$C_{12}$-Cycloalkyl-alkyl, wie Cyclopropyl-methyl, Cyclopentyl-methyl, Cyclopentyl-methyl, Cyclohexyl-methyl, Cycloheptyl-methyl, Cyclooctyl-methyl und 2-Cyclohexylethyl,
- Cyano,
- Hydroxy,
- Hydroxyethyl,
- Phenyl,
- Phenoxy,
- $C_7$-$C_{10}$-Phenalkyl, wie Benzyl, 1-Phenethyl, 2-Phenethyl, 2-Phenpropyl, 3-Phenpropyl und 4-Phenbutyl,
- ein- bis zweifach, bevorzugt einfach, durch $C_1$-$C_4$-Alkyl substituiertes Phenyl, wie 2-Methylphenyl, 3-Methylphenyl, 4-Methylphenyl, 2-Ethylphenyl, 3-Ethylphenyl, 4-Ethylphenyl, 2-n-Propylphenyl, 3-n-Propylphenyl, 4-n-Propylphenyl, 3-iso-Propylphenyl, 4-iso-Propylphenyl, 2-n-Butylphenyl, 3-n-Butylphenyl, 4-n-Butylphenyl, 3-iso-Butylphenyl, 4-iso-Butylphenyl, 3-sec.-Butylphenyl, 4-sec.-Butylphenyl, 3-tert.-Butylphenyl, 4-tert.-Butylphenyl, 2,3-Dimethylphenyl, 2,4-Dimethylphenyl, 2,5-Dimethylphenyl, 3,4-Dimethylphenyl, 3,5-Dimethylphenyl, 2,6-Dimethylphenyl, 3,6-Dimethylphenyl, 2,4-Diethylphenyl, 2,4-Di-iso-propylphenyl, 3,5-Di-tert.-butylphenyl und 2,6-Di-tert.-butylphenyl,
- ein- bis zweifach, bevorzugt einfach, durch $C_1$-$C_4$-Alkoxy substituiertes Phenyl, wie 2-Methoxyphenyl, 3-Methoxyphenyl, 4-Methoxyphenyl, 2-Ethoxyphenyl, 3-Ethoxyphenyl, 4-Ethoxyphenyl, 2-n-Propoxyphenyl, 3-n-Propoxyphenyl, 4-n-Propoxyphenyl, 3-iso-Propoxyphenyl, 4-iso-Propoxyphenyl, 2-n-Butoxyphenyl, 3-n-Butoxyphenyl, 4-n-Butoxyphenyl, 2-sec-Butoxyphenyl, 4-sec-Butoxyphenyl, 2-tert.-Butoxyphenyl, 4-n-Hexoxyphenyl, 2,3-Dimethoxyphenyl, 2,4-Dimethoxyphenyl, 3,4-Dimethoxyphenyl, 2,5-Dimethoxyphenyl, 3,5-Dimethoxyphenyl, 2,6-dimethoxyphenyl und 3,4-Diethoxyphenyl,
- ein- bis zweifach durch Halogen substituiertes Phenyl, bevorzugt durch Fluor oder Chlor, einfach substituiertes Phenyl, wie 2-Fluorphenyl, 2-Chlorphenyl, 2-Bromphenyl, 3-Fluorphenyl, 3-Chlorphenyl, 3-Bromphenyl, 4-Fluorphenyl, 4-Chlorphenyl, 4-Bromphenyl, 2,3-Difluorphenyl, 2,3-Dichlorphenyl, 2,4-Difluorphenyl, 2,4-Dichlorphenyl, 2-Chlor-4-fluorphenyl, 4-Chlor-2-fluorphenyl, 2,5-Difluorphenyl, 2,5-Dichlorphenyl, 3,4-Difluorphenyl, 3,4-Dichlorphenyl, 3,5-Difluorphenyl, 3,5-Dichlorphenyl, 3-Chlor-5-fluorphenyl, 5-Chlor-3-fluorphenyl, 2,6-Difluorphenyl, 2,6-Dichlorphenyl, 2-Chlor-6-fluorphenyl und 6-Chlor-2-fluorphenyl,
- durch $C_1$-$C_4$-Alkyl und $C_1$-$C_4$-Alkoxy substituiertes Phenyl, wie 2-Methyl-4-methoxyphenyl,
- durch $C_1$-$C_4$-Alkyl und Halogen substituiertes Phenyl, wie 2-Methyl-4-chlorphenyl und 3-Methyl-4-fluorphenyl,

- durch $C_1$-$C_4$-Alkoxy und Halogen substituiertes Phenyl, wie 3-Chlor-4-methoxyphenyl,
- ein- bis zweifach durch $C_1$-$C_4$-Alkyl substituiertes Phenoxy, wie 2-Methylphenoxy, 3-Methylphenoxy, 4-Methylphenoxy, 2-Ethylphenoxy, 3-Ethylphenoxy, 4-Ethylphenoxy, 2-n-Propylphenoxy, 3-n-Propylphenoxy, 4-n-Propylphenoxy, 3-iso-Propylphenoxy, 4-iso-Propylphenoxy, 2-n-Butylphenoxy, 3-n-Butylphenoxy, 4-n-Butylphenoxy, 2-sec-Butylphenoxy, 4-sec-Butylphenoxy, 2-tert.-Butylphenoxy, 4-n-Hexylphenoxy, 2,3-Dimethylphenoxy, 2,4-Dimethylphenoxy, 3,4-Dimethylphenoxy, 2,5-Dimethylphenoxy, 3,5-Dimethylphenoxy, 2,6-Dimethylphenoxy und 3,4-Diethylphenoxy,
- ein- bis zweifach durch $C_1$-$C_4$-Alkoxy substituiertes Phenoxy, wie 2-Methoxyphenoxy, 3-Methoxyphenoxy, 4-Methoxyphenoxy, 2-Ethoxyphenoxy, 3-Ethoxyphenoxy, 4-Ethoxyphenoxy, 2-n-Propoxyphenoxy, 3-n-Propoxyphenoxy, 4-n-Propoxyphenoxy, 3-iso-Propoxyphenoxy, 4-iso-Propoxyphenoxy, 2-n-Butoxyphenoxy, 3-n-Butoxyphenoxy, 4-n-Butoxyphenoxy, 3-iso-Butoxyphenoxy, 4-iso-Butoxyphenoxy, 3-sec.-Butoxyphenoxy, 4-sec.-Butoxyphenoxy, 3-tert.-Butoxyphenoxy, 4-tert.-Butoxyphenoxy, 2,3-Dimethoxyphenoxy, 2,4-Dimethoxyphenoxy, 2,5-Dimethoxyphenoxy, 3,4-Dimethoxyphenoxy, 3,5-Dimethoxyphenoxy, 2,6-Dimethoxyphenoxy, 3,6-Dimethoxyphenoxy, 2,4-Diethoxyphenoxy, 2,4-Di-iso-propoxyphenoxy, 3,5-Di-tert.-butoxyphenoxy und 2,6-Di-tert.-butoxyphenoxy,
- ein- bis zweifach durch Halogen substituiertes Phenoxy, bevorzugt durch Fluor und/oder Chlor, einfach substituiertes Phenoxy, wie 2-Fluorphenoxy, 2-Chlorphenoxy, 3-Fluorphenoxy, 3-Chlorphenoxy, 4-Fluorphenoxy, 4-Chlorphenoxy, 2,3-Difluorphenoxy, 2,3-Dichlorphenoxy, 2,4-Difluorphenoxy, 2,4-Dichlorphenoxy, 2-Chlor-4-fluorphenoxy, 4-Chlor-2-fluorphenoxy, 2,5-Difluorphenoxy, 2,5-Dichlorphenoxy, 3,4-Difluorphenoxy, 3,4-Dichlorphenoxy, 3,5-Difluorphenoxy, 3,5-Dichlorphenoxy, 3-Chlor-5-fluorphenoxy, 5-Chlor-3-fluorphenoxy, 2,6-Difluorphenoxy, 2,6-Dichlorphenoxy, 2-Chlor-6-fluorphenoxy und 6-Chlor-2-fluorphenoxy,
- durch $C_1$-$C_4$-Alkyl und $C_1$-$C_4$-Alkoxy substituiertes Phenoxy, wie 2-Methyl-4-methoxyphenoxy,
- durch $C_1$-$C_4$-Alkyl und Halogen substituiertes Phenoxy, wie 2-Methyl-4-chlorphenoxy und 3-Methyl-4-fluorphenoxy,
- durch $C_1$-$C_4$-Alkyl und Halogen substituiertes Phenoxy, wie 3-Chlor-4-methoxyphenoxy,
- ein- bis zweifach durch $C_1$-$C_4$-Alkyl substituiertes $C_7$-$C_{10}$-Phenalkyl wie 4-Methylbenzyl und 3,4-Dimethylbenzyl,
- ein- bis zweifach durch $C_1$-$C_4$-Alkoxy substituiertes $C_7$-$C_{10}$-Phenalkyl wie 4-Methylbenzyl und 3,4-Dimethoxybenzyl,
- ein- bis zweifach durch Halogen substituiertes $C_7$-$C_{10}$-Phenalkyl, bevorzugt durch Fluor oder Chlor ein- bis zweifach substituiertes $C_7$-$C_{10}$-Phenalkyl, wie 4-Fluorbenzyl und 4-Chlorbenzyl,

$R^4$

- Wasserstoff,
- eine Sulfonsäuregruppe.

Im Hinblick auf die Verwendung der 4-Alkoxy-2-hydroxybenzophenon-5-sulfonsäuren I als Lichtschutzmittel sind diejenigen Reste besonders bevorzugt, in denen $R^1$ für eine $C_1$-$C_{18}$-Alkylgruppe, $R^2$ in ortho-Stellung zur ketonische Carbonylgruppe steht und Wasserstoff oder eine Hydroxygruppe darstellt und $R^4$ für Wasserstoff steht. Weiterhin sind von Bedeutung Verbindung I, in denen $R^1$ für ein $C_1$-$C_{18}$-Alkylgruppe, $R^2$ und/oder $R^3$ für Wasserstoff oder $R^2$ für eine ortho-Hydroxygruppe und $R^3$ für eine para-Alkoxygruppe und $R^4$ für Wasserstoff stehen.

Im allgemeinen fallen die 4-Alkoxy-2-hydroxybenzophenon-5-sulfonsäuren I bereits in ausreichender Reinheit an, es kann jedoch ein weiterer Reinigungsschritt auf einfache Weise mit der Aufarbeitung verbunden werden, derart, daß man beispielsweise der oben beschriebenen wäßrigen Lösung Aktivkohle zusetzt, filtriert und die Verbindungen I aus dem Filtrat in der obenbeschriebenen Weise isoliert. Man kann auch eine Nachreinigung z.B. durch Umfällen aus wäßriger Lösung oder Ausrühren bzw. Waschen mit einem Lösungsmittel durchführen.

Je nach Aufarbeitung können die hygroskopischen 4-Alkoxy-2-hydroxybenzophenon-5-sulfonsäuren I einen Wassergehalt von 0,01 bis 20 Gew.%, vorzugsweise von 0,5 bis 15 Gew.% enthalten. Der Wassergehalt ist für die Anwendung nicht störend.

Die 4-Alkoxy-2-hydroxybenzophenon-5-sulfonsäuren I eignen sich beispielsweise als Lichtschutzmittel, z.B. in kosmetischen Präparaten.

Im Hinblick auf ihre Verwendung als Lichtschutzmittel besonders geeignete Verbindungen I sind:
2-Hydroxy-4-methoxybenzophenon-5-sulfonsäure,
2-Hydroxy-4-ethoxybenzophenon-5-sulfonsäure,
2-Hydroxy-4-n-propoxybenzophenon-5-sulfonsäure,
2-Hydroxy-4-iso-propoxybenzophenon-5-sulfonsäure,
2-Hydroxy-4-n-butoxybenzophenon-5-sulfonsäure,

2-Hydroxy-4-iso-butoxybenzophenon-5-sulfonsäure,
2-Hydroxy-4-sec-butoxybenzophenon-5-sulfonsäure,
2-Hydroxy-4-n-pentoxybenzophenon-5-sulfonsäure,
2-Hydroxy-4-n-hexoxybenzophenon-5-sulfonsäure,
2-Hydroxy-4-iso-hexoxybenzophenon-5-sulfonsäure,
2-Hydroxy-4-n-heptoxybenzophenon-5-sulfonsäure,
2-Hydroxy-4-iso-heptoxybenzophenon-5-sulfonsäure,
2-Hydroxy-4-n-octoxybenzophenon-5-sulfonsäure,
2-Hydroxy-4-(3,4-dimethyl-1-hexoxy)benzophenon-5-sulfonsäure,
2-Hydroxy-4-(3,5-dimethyl-1-hexoxy)benzophenon-5-sulfonsäure,
2-Hydroxy-4-(4,5-dimethyl-1-hexoxy)benzophenon-5-sulfonsäure,
2-Hydroxy-4-(3-methyl-1-heptoxy)benzophenon-5-sulfonsäure,
2-Hydroxy-4-n-nonoxybenzophenon-5-sulfonsäure,
2-Hydroxy-n-decoxybenzophenon-5-sulfonsäure,
2-Hydroxy-4-n-undecoxybenzophenon-5-sulfonsäure,
2-Hydroxy-4-n-dodeoxybenzophenon-5-sulfonsäure,
2-Hydroxy-4-n-hexadecoxybenzophenon-5-sulfonsäure,
2-Hydroxy-4-n-octadecoxybenzophenon-5-sulfonsäure,
2-Hydroxy-4-(2-acetoxyethoxy)benzophenon-5-sulfonsäure,
2-Hydroxy-4-(2-phenbenzoyloxyethoxy)benzophenon-5-sulfonsäure,
2-Hydroxy-4-phenylmethylenoxybenzophenon-5-sulfonsäure,
2-Hydroxy-4-(2-phenylethylenoxy)benzophenon-5-sulfonsäure,
2,2′-Dihydroxy-4,4′-dimethoxy-5-sulfonsäure,
2,2′-Dihydroxy-4,4′-dimethoxybenzophenon-5,5′-disulfonsäure,
2,2′-Dihydroxy-4-methoxybenzophenon-5-sulfonsäure,
2-Hydroxy-4′-fluor-4-methoxybenzophenon-5-sulfonsäure,
2-Hydroxy-4-methoxy-4′-methylbenzophenon-5-sulfonsäure,
2-Hydroxy-4-methoxy-4′-phenoxybenzophenon-5-sulfonsäure,
2-Hydroxy-4-methoxy-4′-chlorbenzophenon-5-sulfonsäure,
2-Hydroxy-4-methoxycarbonylmethylenoxybenzophenon-5-sulfonsäure,
2-Hydroxy-4-ethoxycarbonylmethylenoxybenzophenon-5-sulfonsäure.

Beispiele

Herstellung von 2-Hydroxy-4-methoxybenzophenon-5-sulfonsäure (Beispiel 1 bis 3)

Beispiel 1

In 60 ml Essigsäureethylester trug man 22,8 g 2-Hydroxy-4-methoxybenzophenon ein, tropfte unter Kühlung (ca. 0 bis 5°C Innentemperatur) innerhalb von 30 Minuten 31 g Chlorsulfonsäure zu und rührte über Nacht bei Raumtemperatur (18 bis 25°C). Anschließend kühlte man auf 10°C, saugte das Produkt ab, wusch mit 10 ml Essigsäureethylester nach und trocknete bei 30°C i. Vak. Man erhielt 26 g 2-Hydroxy-4-methoxybenzophenon-5-sulfonsäure; Fp.: 193 bis 195°C mit einem Wassergehalt von 3,7 Gew.%.

Beispiel 2

22,8 g (0,1 mol) 2-Hydroxy-4-methoxybenzophenon wurden bei Raumtemperatur in einer Mischung aus 43 ml des Filtrats von Beispiel 1 und 17 ml Essigsäureethylester gelöst analog Beispiel 1 umgesetzt, wobei man nach Trocknung bei 30 bis 40°C i.Vak. 27 g 2-Hydroxy-4-methoxybenzophenon-5-sulfonsäure erhielt; Fp.: 196 bis 198°C (Wassergehalt 2,5 Gew.%).

Beispiel 3

In 30 ml Benzoesäureethylester löste man bei Raumtemperatur 11,4 g 2-Hydroxy-4-methoxybenzophenon, setzte eine Spatelspitze 2-Hydroxy-4-methoxybenzophenon-5-sulfonsäure zu und tropfte unter Außenkühlung innerhalb von 30 Minuten 3,5 ml Chlorsulfonsäure zu. Nach etwa 20 Minuten setzte Kristallisation ein. Man rührte noch 4 Stunden bei Raumtemperatur nach, kühlte auf 10°C und saugte ab. Man wusch zweimal mit wenig Essigsäuremethylester, trocknete i. Vak. bei 30°C und erhielt 13,5 g 2-Hydroxy-4-methoxybenzophenon-5-sulfonsäure; Fp.: 188 bis 190°C (Wassergehalt <0,5 Gew.%).

Beispiel 4

Herstellung von 2,2′-Dihydroxy-4,4′-dimethoxybenzophenon-5-sulfonsäure

Zu einer Suspension von 13,7 g (0,05 mol) 2,2′-Dihydroxy-4,4′-dimethoxybenzophenon in 50 ml Benzoesäureethylester tropfte man bei 20°C 3,5 ml (0,05 mol) Chlorsulfonsäure. Anschließend wurde 4 Stunden bei 20°C gerührt, der Niederschlag abgesaugt, zweimal mit je 10 ml Essigsäureethylester gewaschen und mit 30 ml Diethylether angerührt. Nach Trocknen i. Vak. bei 30°C erhielt man 9,1 g 2,2′-Dihydroxy-4,4′-dimethoxybenzophenon-5-sulfonsäure; Fp.: 151 bis 153°C (Wassergehalt 2,4 Gew.%).

Beispiel 5

In 30 ml Phthalsäuredimethylester löste man bei 80 °C 11,4 g 2-Hydroxy-4-methoxybenzophenon und tropfte bei dieser Temperatur 3,5 ml Chlorsulfonsäure zu. Man rührte noch 4 Stunden bei 80 °C nach, kühlte auf Raumtemperatur ab und versetzte die Lösung mit 25 ml Ether. Der Niederschlag wurde abgesaugt, mit Ether und Hexan gewaschen und im Vakuum bei 30 °C getrocknet. Man erhielt 10 g 2-Hydroxy-4-methoxybenzophenon-5-sulfonsäure vom Schmelzpunkt 112 bis 114 °C (Wassergehalt 7,7 %).

Beispiel 6

In 25 ml Benzoesäureethylester löste man bei Raumtemperatur 6,56 g 2-Hydroxy-4-methoxy-4′-chlorbenzophenon und tropfte 1,75 ml Chlorsulfonsäure zu. Nach vierstündigem Rühren bei Raumtemperatur goß man das Reaktionsgemisch auf 50 ml Wasser, trennte die Wasserphase ab und dampfte sie im Vakuum (ca. 10 mbar) ein. Der Rückstand wurde mit ca. 30 ml Ether verrührt, abgesaugt, mit Ether gewaschen und im Vakuum bei 30 °C getrocknet. Man erhielt 7,4 g 2-Hydroxy-4-methoxy-4′-chlorbenzophenon-5-sulfonsäure vom Schmelzpunkt 123 bis 125 °C (Wassergehalt 9,5 %).

Beispiel 7

In 15 ml Benzolsäureethylester löste man bei Raumtemperatur 8 g 2-Hydroxy-4-methoxy-4′-phenoxybenzophenon und tropfte 1,75 ml Chlorsulfonsäure zu. Nach zwölfstündigem Rühren bei Raumtemperatur wurde das pastöse Reaktionsgemisch mit 30 ml Ether verrührt, der Rückstand abgedampft, mit Ether gewaschen und im Vakuum bei 30 °C getrocknet. Man erhielt 4,3 g 2-Hydroxy-4-methoxy-4′-phenoxybenzophenon-5-sulfonsäure vom Schmelzpunkt 169 bis 171 °C (Wassergehalt 1,2 %).

Beispiel 8

In 25 ml Benzoesäureethylester löste man bei Raumtemperatur 6,45 g 2-Hydroxy-4,4′-dimethoxybenzophenon und tropfte 1,75 ml Chlorsulfonsäure zu. Man verfuhr weiter analog Beispiel 6 und erhielt 7,3 g 2-Hydroxy-4,4′-dimethoxybenzophenon-5-sulfonsäure vom Schmelzpunkt 125 bis 127 °C (Wassergehalt 10,5 %).

Beispiel 9

Zu einer Suspension von 13,7 g 2,2′-Dihydroxy-4,4′-dimethoxybenzophenon in 50 ml Benzoesäureethylester tropfte man bei Raumtemperatur 3,5 ml Chlorsulfonsäure. Im Laufe des Zutropfens entstand vorübergehend eine klare Lösung, aus der während des vierstündigen Nachrührens bei Raumtemperatur ein Niederschlag ausfiel. Nach Abkühlen auf 10°C saugte man ab und wusch zweimal mit je 10 ml kaltem (ca. 10 °C) Essigsäureethylester nach. Der Rückstand wurde noch zweimal mit je 30 ml Ether verrührt, abgesaugt und im Vakuum bei 30 °C getrocknet. Man erhielt 7 g 2,2′-Dihydroxy-4,4′-dimethoxybenzophenon-5-sulfonsäure vom Schmelzpunkt 160 bis 162 °C (Wassergehalt 1,7 %).

Beispiel 10

Man löste 21,3 g 2-Hydroxy-4-n-octoxybenzophenon bei Raumtemperatur in 30 ml Benzoesäureethylester und tropfte 3,5 ml Chlorsulfonsäure zu. Nach vierstündigem Rühren bei Raumtemperatur kühlte man auf 10

°C, saugte den Niederschlag ab und wusch mit 10 ml Essigsäureethylester nach. Der Rückstand wurde mit 10 ml Hexan ausgerührt und im Vakuum getrocknet. Man erhielt 3,6 g 2-Hydroxy-4-n-octoxybenzophenon-5-sulfonsäure vom Schmelzpunkt 165 bis 167 °C (Wassergehalt 4,4 %). Nach Versetzen der verbliebenen Benzoesäureethylesterlösung mit 40 ml Hexan erhielt man weitere 13 g der Sulfonsäure.

Beispiel 11

Man suspendiert 2,8 g 2-Hydroxy-4-methoxycarbonylmethylenoxybenzophenon bei Raumtemperatur in 10 ml Benzoesäureethylester und tropfte 0,7 ml Chlorsulfonsäure zu. Nach vierstündigem Nachrühren bei Raumtemperatur wurde abgesaugt und zweimal mit je ca. 5 ml Essigsäureethylester gewaschen. Nach Ausrühren des Rückstands mit ca. 10 ml Ether und Trocknen im Vakuum bei 30 °C erhielt man 1,9 g der 2-Hydroxy-4-methoxy-carbonylmethylenoxybenzophenon-5-sulfonsäure vom Schmelzpunkt 145 bis 146 °C (Wassergehalt 4,5 %).

Beispiel 12

In 400 ml Essigsäureethylester trug man 114 g 2-Hydroxy-4-methoxybenzophenon ein, tropfte unter Kühlung bei einer Innentemperatur von 20°C 40 ml Chlorsulfonsäure innerhalb 45 min zu und rührte 4 Stunden bei 20 bis 25°C. Anschließend kühlte man auf 15°C ab, saugte das Produkt ab, wusch mit 60 ml Heptan, trocknete i.Vak. bei 50°C und erhielt 133 g 2-Hyroxy-4-methoxybenzophenon-5-sulfonsäure; Fp.: 195 bis 196°C, Wassergehalt: <0,1 %.

Durch Zugabe von 15 ml 70 %iger Schwefelsäure zum Filtrat konnten weitere 8,3 g des Produkts vom Fp.: 160 bis 162°C mit einem Wassergehalt von 2,4 % ausgefällt werden.

## Patentansprüche

1. Verfahren zur Herstellung von 4-Alkoxy-2-hydroxybenzophenon-5-sulfonsäuren der allgemeinen Formel I

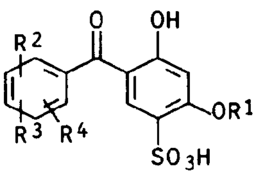

(I),

in der

$R^1$ $C_1$-$C_{20}$-Alkyl, das gegebenenfalls durch Halogen, Cyano, Hydroxy, $C_1$-$C_{20}$-Alkoxy, $C_1$-$C_{20}$-Alkoxycarbonyl, Acyloxy und/oder gegebenenfalls durch $C_1$-$C_4$-Alkyl, $C_1$-$C_4$-Alkoxy und/oder Halogen substituiertes Phenyl, substituiert ist,

$R^2$, $R^3$ unabhängig voneinander Wasserstoff, Halogen, $C_1$-$C_{12}$-Alkyl, $C_1$-$C_{12}$-Alkoxy, $C_1$-$C_4$-Halogenalkyl, $C_3$-$C_8$-Cycloalkyl, $C_4$-$C_{12}$-Cycloalkyl-alkyl, Cyano, Hydroxy, Hydroxyethyl oder gegebenenfalls durch $C_1$-$C_4$-Alkyl, $C_1$-$C_4$-Alkoxy und/oder Halogen substituiertes Phenyl, Phenoxy oder $C_7$-$C_{10}$-Phenalkyl und

$R^4$ Wasserstoff oder eine Sulfonsäuregruppe

bedeuten, durch Umsetzung eines 4-Alkoxy-2-hydroxybenzophenons der allgemeinen Formel II

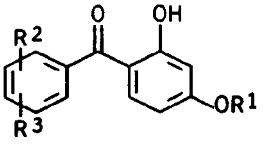

(II),

mit Chlorsulfonsäure bei Temperaturen von (-20) bis +100°C, dadurch gekennzeichnet, daß man die Umsetzung in einem aliphatischen, cycloaliphatischen und/oder aromatischen Carbonsäureester als Lösungsmittel vornimmt.

## Claims

1. A process for the preparation of a 4-alkoxy-2-hydroxybenzophenone-5-sulfonic acid of the formula I

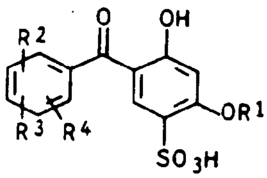

(I)

where $R^1$ is $C_1$-$C_{20}$-alkyl which is unsubstituted or substituted by halogen, cyano, hydroxyl, $C_1$-$C_{20}$-alkoxy, $C_2$-$C_{20}$-alkoxycarbonyl, acyloxy and/or phenyl which is unsubstituted or substituted by $C_1$-$C_4$-alkyl, $C_1$-$C_4$-alkoxy and/or halogen,
$R^2$ and $R^3$ independently of one another are each hydrogen, halogen, $C_1$-$C_{12}$-alkyl, $C_1$-$C_{12}$-alkoxy, $C_1$-$C_4$-haloalkyl, $C_3$-$C_8$-cycloalkyl, $C_4$-$C_{12}$-cycloalkylalkyl, cyano, hydroxyl or hydroxyethyl or are each phenoxy, $C_7$-$C_{10}$-phenalkyl or phenyl which is unsubstituted or substituted by $C_1$-$C_4$-alkyl, $C_1$-$C_4$-alkoxy and/or halogen, and $R^4$ is hydrogen or sulfo, by reacting a 4-alkoxy-2-hydroxybenzophenone of the formula II

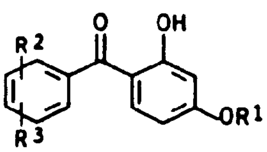

(II)

with chlorosulfonic acid at from -20 to +100°C, wherein the reaction is carried out in an aliphatic, cycloaliphatic and/or aromatic carboxylate as solvent.

## Revendications

1. Procédé de préparation d'acides 4-alcoxy-2-hydroxybenzophénone-5-sulfoniques de formule générale I

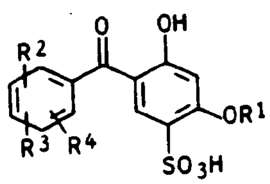

(I),

dans laquelle

$R^1$ représente un reste alkyle en $C_1$-$C_{20}$ qui est éventuellement substitué par un atome d'halogène ou un radical cyano, hydroxy, alcoxy en $C_1$-$C_{20}$, (alcoxy en $C_1$-$C_{20}$)carbonyle, acyloxy et/ou phényle éventuellement substitué par un radical alkyle en $C_1$-$C_4$, alcoxy en $C_1$-$C_4$ et/ou un atome d'halogène,

$R^2$, $R^3$ représentent chacun, indépendamment l'un de l'autre, un atome d'hydrogène, d'halogène, un reste alkyle en $C_1$-$C_{12}$, alcoxy en $C_1$-$C_{12}$, halogéno-alkyle en $C_1$-$C_4$, cycloalkyle en $C_3$-$C_8$, cycloalkylalkyle en $C_4$-$C_{12}$, cyano, hydroxy, hydroxyéthyle ou phényle éventuellement substitué par un radical alkyle en $C_1$-$C_4$, alcoxy en $C_1$-$C_4$ et/ou un atome d'halogène, un reste phénoxy ou phénalkyle en $C_7$-$C_{10}$, et

$R^4$ représente un atome d'hydrogène ou un groupement acide sulfonique,

par réaction d'une 4-alcoxy-2-hydroxybenzophénone de formule générale II

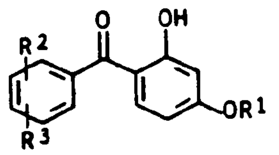

(II),

avec de l'acide chlorosulfonique à des températures de -20 à +100°C, caractérisé en ce qu'on conduit la réaction dans un ester d'acide carboxylique aliphatique, cycloaliphatique et/ou aromatique en tant que solvant.